# DEMANDE DE BREVET EUROPEEN

(11) **EP 4 674 468 A1**
(43) Date de publication de la demande: **07.01.2026**
(21) Numéro de dépôt: 24315333.5
(22) Date de dépôt: 05.07.2024
(51) Int. Cl.: A61N 1/04, A61N 1/36, A61N 1/32

(54) **DISPOSITIF D'ÉLECTROSTIMULATION AGENCÉ POUR L'ÉLECTROSTIMULATION SUR UN UTILISATEUR ET INSTALLATION**

(71) Demandeur: Genhealth Corporation SAS, 57100 Thionville (FR)
(72) Inventeur: DELABAS, Olivier, 54510 Tomblaine (FR)
(74) Mandataire: Munier, Laurent

(57) **Abrégé**

|Le système comprend le dispositif d'électrostimulation (100) qui comporte une pluralité d'électrodes (1), un générateur (10) contrôlé par un calculateur (20) et des moyens logiciels (30). Ledit calculateur (20) comporte au moins une mémoire (40) comportant des données relatives audit utilisateur. Ledit générateur (10) comporte une pluralité de voies excitomotrices (11), chacune rattachée à un câble (12) porteur d'une pluralité de dites électrodes (1), agencée pour commander l'envoi de signaux d'excitation aux différentes voies excitomotrices (11).

Le dispositif d'électrostimulation (100) est agencé pour l'électrostimulation sur un utilisateur de forte corpulence.

Les Installations comportent un dispositif d'électrostimulation (100).

## Description

[La présente invention concerne un moyen de maintien de la masse musculaire pour un utilisateur de forte corpulence durant la phase de restriction calorique.

Traditionnellement, plusieurs moyens sont connus en vue perte de du poids, des procédés chirurgicaux, comme une liposuccion, la prise de médicaments, souvent médicaments anti-diabétiques les exercices avec les dispositifs de salle de musculation, l'utilisation d'électrodes.

Ces moyens présentent des limitations ou des inconvénients.

Une liposuccion provoque un relâchement cutané, des bleus pendant trois semaines. C'est un moyen dévastateur pour le corps. Outre ces inconvénients, la liposuccion présente la limitation d'être inefficace à terme. En effet, elle est suivie après un certain temps au retour du poids d'origine.

Les médicaments anti-diabétiques. Ils ont été développés pour le traitement du diabète de type 2, le médicament dit Ozempic peut grandement améliorer le quotidien des personnes aux prises avec cette maladie. C'est toutefois un autre effet qui retient l'attention du public et des pharmaceutiques. En ralentissant la vitesse à laquelle l'estomac se vidange et en agissant directement sur la sensation d'être rassasié. Selon une étude publiée en 2021 dans le New England Journal of Medicine, l'Ozempic a permis une perte de poids moyenne de 14,9 % chez des patientes et des patients en surpoids ou obèses qui ont également changé leurs habitudes de vie. Malheureusement, une telle perte de poids engendre une perte de masse musculaire, une perte progressive de la masse et de la force musculaires qui survient avec le vieillissement. C'est pourquoi il est essentiel d'avoir un apport suffisant en protéines et un entraînement musculaire régulier en parallèle de la perte de poids.

Ce moyen entre dans la catégorie de baisse de l'apport calorique et présente des inconvénients de la même nature que des régimes alimentaires et la bonne alimentation.

Une question se pose ; que veut dire bien manger ? Il n'existe pas de vraie définition d'une bonne alimentation. L'exemple universel d'hétérogénéité alimentaire est celui du petit déjeuner.

Toutefois, bien manger ; c'est manger le moins transformé. Cela nécessité de cuisiner. La baisse calorique est mesurable.

Il est nécessaire de prévenir le risque de dénutrition.

L'âge, la maladie ou encore les traitements peuvent induire une perte de l'appétit ou du goût, entraînant un risque de dénutrition. Une perte de poids et une diminution de la masse musculaire peuvent alors apparaître. Pour éviter tout risque de dénutrition, il est important de maintenir une alimentation suffisante répartie sur trois repas quotidiens.

L'origine de ces incontinents vient de ce que la masse musculaire protège des maladies. Elle limite l'impact de la mauvaise alimentation.

Ainsi, une diminution en chute libre de la masse musculaire est pathogène.

Autre limitation, si l'amaigrissement est rapide la peau s'effondre (effet tablier).

L'alimentation seule ne permet pas d'obtenir durablement une perte de poids.

Pour cela une alimentation équilibrée doit être combinée à une activité musculaire souvent une activité physique régulière, pour permettre de prévenir le surpoids et d'éviter plusieurs affections ou risques plus fréquents avec l'âge. C'est le cas du diabète, de l'hypertension artérielle, de l'hypercholestérolémie, de l'ostéoporose, ou encore des troubles de la mémoire ou de la constipation.

Pour avoir l'activité physique régulière l'utilisateur connaît les salles de musculation.

Une salle de musculation présente les caractéristiques et limitations suivantes :
• Equipement et agrès pour faire travailler les muscles.
• Pas d'équilibre par muscle agoniste et antagoniste : risque de blessure.
• Nécessite de nombreuses répétitions et une fréquence importante.
• L'intensité est nécessaire, mais peu contrôlable et quelques fois impossible selon l'état physique et physiologique du patient.
• Pas de constance dans la durée et la répétition : risque de démotivation lié à la fatigue et perte d'efficacité, accumulation de lactate importante.

Des travaux récents suggèrent qu'il est utile de bien distinguer les deux phases de traitement de l'excès de poids (la phase de perte de poids et la phase de stabilisation pondérale) en termes de moyen thérapeutiques (diététique et activité physique).

Les stratégies de perte de poids sont assez bien maitrisées alors que la stabilisation pondérale après amaigrissement reste un problème difficile.

Cependant, la recherche a montré que 20% de personnes en surpoids ont réussi à perdre du poids à long terme lorsqu'elle est définie comme une perte d'au moins 10% du poids corporel initial et pour un maintien de la perte d'au moins 1 an. (Wing & Phelan, 2005).

Les stratégies de prise en charge sont les plus efficaces quand elles sont multifactorielles et agissent à plusieurs niveaux de comportements. Il apparait actuellement que l'association d'une restriction calorique et d'une augmentation du niveau d'activité physique est celle qui semble la plus efficace sur le maintien d'une perte de poids à long terme (Stiegler & Cunliffe, 2006). Sur le cours terme, parmi ces 2 facteurs, la restriction calorique reste parmi ces deux interventions la plus efficaces pour corriger l'excès de masse grasse, mais l'exercice musculaire permet le maintien de la masse maigre, favorisant ainsi le maintien à long terme de la perte de poids. (Curioni, Lourenço, Int J Obes (Lond) 2005).

Il faut comprendre que la restriction calorique entraine une fonte musculaire allant jusqu'à 30% du poids total, contre 12 % si un régime est associé à un programme d'endurance, (Ballor et Poehlman 1994) et surtout contre 0% si une combinaison restriction calorique et activité physique aérobie-musculation s'effectue conjointement. (Kraemer et al.)

Malgré un consensus sur les modalités efficaces de l'activité physique pour le maintien de la perte de poids à long terme (consensus pour 45-60 minutes par jour d'intensité modérée soit entre 3 et 6 METS, versus 30 minutes de marche rapide par jour (Saris, 2003), il existe des éléments dans la littérature qui suggèrent que des programmes d'exercice plus intenses induisent des bénéfices plus importants, en effet, le rôle de l'intensité est à privilégier plutôt que le volume d'activité physique. (Gutin et al. 2002).

La restriction calorique doit s'accompagner d'un développement musculaire. Ce qui demande une intensité, soit une activité sportive d'endurance musculaire (aérobie), du cardio. Mais gainer n'est pas se muscler,

En outre, il est important de souligner les diverses contraintes liées à la surcharge pondérale et l'obésité dans le cadre d'une pratique d'activité sportive et du développement musculaire. En effet, les difficultés à se mouvoir, la fatigue accrue, les essoufflements, les complications cardiovasculaires, respiratoires, métaboliques, ostéoarticulaires et rhumatismales sont autant de facteurs accrus empêchant la pratique d'une activité physique continue, tout en rajoutant la perturbation de l'image du corps et donc d'estime de soi, posant la question de l'utilisation de nouveaux outils de substitution à l'activité physique.

Ainsi, compte tenu des complications cardiovasculaires, respiratoires et ostéo-articulaires des patients en surpoids ou en obésité dans la reprise d'une activité physique et de son importance dans le cadre du maintien de la masse musculaire, il semble intéressant de pouvoir y apporter une assistance physique capable de reproduire des effets similaires à l'APS (activité physique et sportive) comme le ferait l'électrostimulation (Banerjee et al. 2005)37 sur la dépense énergétique (Hsu, Wei, Chang. 2011).

La masse musculaire est mesurable.

L'invention a pour but de surmonter ces inconvénients. La base de l'invention réside dans le fait que malgré les progrès de la science, il n'existe pas à l'heure actuelle d'appareil capable de prendre en charge de façon globale, rapide et durable, la recapitalisation musculaire. Les dispositifs de l'état de la techniques (connaissance interne de la déposante) sont d'une conception ne permettant pas une prise en charge efficace notamment des personnes en surpoids ou sur certaines parties du corps. De plus, ces dispositifs restent d'une technologie complexe qui entraîne des coûts largement augmentés.

L'invention concerne un système comprenant des électrodes d'électrostimulation dites EMS intégral ou corps entier, leur moyen d'alimentation en particulier l'alimentation directe et la mise en oeuvre du procédé d'utilisation du système d'électrostimulation.

En premier lieu, l'invention se rapporte à un dispositif d'électrostimulation.

La présente invention a pour but une rupture d'ampleur dans la visée du maintien de santé et prévention de la fonte musculaire liée à l'avancée en âge. Elle rend accessible et efficace la prise en charge de personnes en surpoids durant leur phase de restriction calorique ce qui concerne un nombre important de personnes en utilisant une combinaison inédite d'ultrasons et d'EMS Stabile.

L'électrostimulation est le principe d'avoir des électrodes sur l'ensemble des muscles qui vont se contracter involontairement mais régulièrement et efficacement. Elle mobilise plus de 400 muscles en même temps pendant 20 minutes, aucun mouvement selon niveau (pompe, gainage, squats, etc.).

Pour les électrodes l'invention passe en silicone et surtout "électrodes type" en TPE conducteur ; modèle avec électrodes à taille unique, modèle RFID avec tailles d'électrodes et logiciel intégré.

L'invention a pour but d'obtenir 100% de la contraction du groupe musculaire. Pour cela il est :
- placé une électrode par groupe musculaire/à la morphologie,
- obtenu un travail simultané des muscles agonistes et antagonistes,
- une vague d'impulsion : courant excitomoteur + courant anti-douleur (tens),
- utilisé les électrodes connectées : connaissance de la puissance délivrée propre à la S² de l'électrode,
- utilisé des montages d'électrodes adaptés aux personnes en surpoids ou en obésité.

Le tableau ci-dessous présente le design et caractéristiques des électrodes EMS selon l'invention :

| Désignation | Dimensions | Superficie | Masse (estimative) |
|---|---|---|---|
| (Largeur x hauteur x épaisseur) | | | |
| HTC-ELEC-192 | 160 x 120 x 2 mm | 192 mm² | 50 g |

L'électrostimulation est mesurable dans sa fréquence et son amplitude. Il est recherché des vagues d'impulsions avec des amplitudes les plus haute possible.

Une amplitude à 85 Hz des possibilités de la machine est efficace dans le développement musculaire et la conversion des fibres musculaires.

En premier lieu, l'invention se rapporte à un dispositif d'électrostimulation agencé pour l'électrostimulation sur un utilisateur de forte corpulence, ayant un indice de masse corporelle supérieur à 26 à 32 kg/m², un tour de taille supérieur à 108 cm, une masse supérieure à 100 kg, ledit dispositif d'électrostimulation comportant une pluralité d'électrodes.

Pour résoudre les problèmes techniques ci-dessus le dispositif d'électrostimulation selon l'invention comporte un générateur contrôlé par un calculateur et des moyens logiciels, ledit calculateur comportant au moins une mémoire comportant des données relatives audit utilisateur prédéfinies par un examen préalable, ledit générateur comportant une pluralité de voies excitomotrices, chacune rattachée à un câble porteur d'une pluralité de dites électrodes, et étant agencée pour commander l'envoi de signaux d'excitation simultanés ou séparés aux différentes voies excitomotrices.

Suivant d'autres caractéristiques dudit dispositif d'électrostimulation :
- chaque dit câble a une longueur minimale de 120 à 150 cm ;
- au moins une dite électrode comporte une enveloppe en élastomère thermoplastique conducteur, qui comporte au moins une puce RFID agencée pour l'identification de ladite électrode ;

L'invention a encore pour objet une installation de culture physique, comportant au moins un agrès ou une machine d'exercice musculaire ou un tapis d'exercice, et comportant au moins un dispositif d'électrostimulation selon au moins l'une des caractéristiques décrites ci-dessus.

L'invention concerne encore pour objet une installation de détente, comportant un siège ou une couche pour supporter l'utilisateur, et comportant au moins un dispositif d'électrostimulation selon au moins l'une des caractéristiques décrites ci-dessus.

L'invention sera mieux comprise à la lecture des figures données à titre d'exemples non limitatifs et sur lesquelles :

La Fig. 1 représente schématiquement vu de dessus un dispositif d'électrostimulation selon l'invention comprenant une structure porteuse, un générateur, des moyens logiciels, des câbles et des électrodes d'électrostimulation.

La Fig. 2 représente vu de face et ¾ dessus un boitier, appareil du dispositif d'électrostimulation selon l'invention comprenant un générateur comportant une pluralité de voies excitomotrices avec huit moyens de connexion à des câbles porteur de dites électrodes, comportant quatre moyens de réglage de l'intensité.

La Fig. 3 représente vu en élévation le boitier appareil du dispositif d'électrostimulation selon l'invention comportant huit moyens de connexion à des câbles et comportant quatre moyens de réglage de l'intensité et une antenne dite Bluetooth.

La Fig. 4 représente vu de dessus l'enveloppe en élastomère thermoplastique conducteur d'une électrode d'électrostimulation qui comprend une puce RFID.

La Fig. 5 représente vu en élévation le système avec dispositif comprenant des électrodes d'électrostimulation identiques et intervertibles branchées par deux sur des câbles selon l'invention en fonctionnement dans une variante d'emplacement des électrodes sur l'utilisateur.

La Fig. 6 est un schéma fonctionnel des mesures effectuées avec un appareil de référence lors des essais de l'invention.

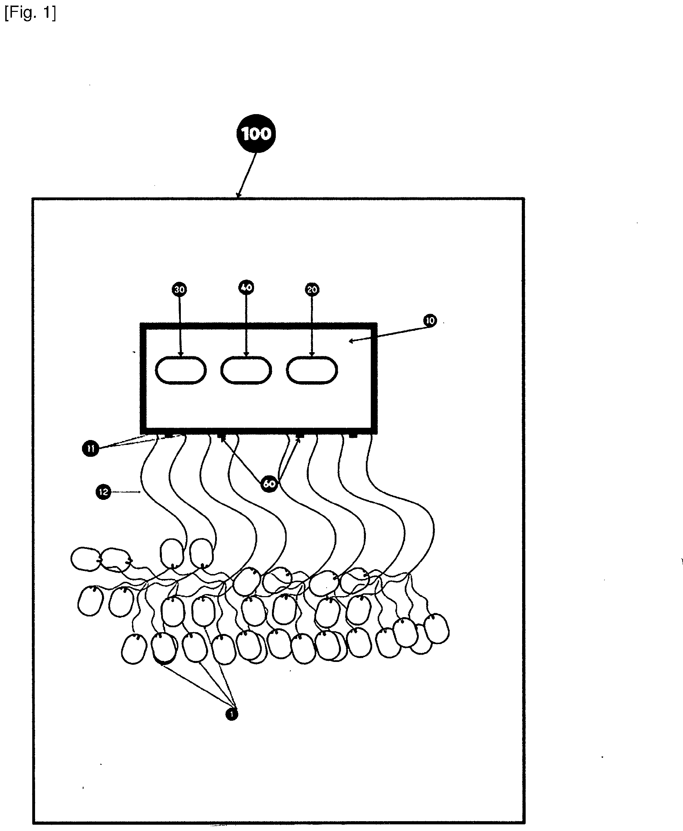

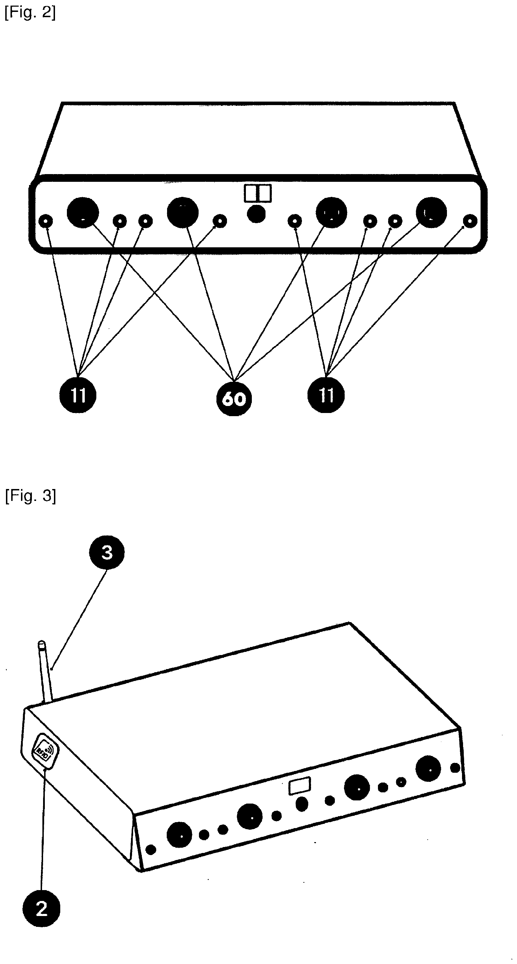

Les Figures 1 à 5 concernant un appareil, elles représentent un dispositif d'électrostimulation 100 agencé pour l'électrostimulation sur un utilisateur de forte corpulence, ayant un indice de masse corporelle supérieur à 26 à 32 kg/m², un tour de taille supérieur à 108 cm, une masse supérieure à 100 kg. Ledit dispositif d'électrostimulation 100 comporte une pluralité d'électrodes 1. Les électrodes 1 sont une enveloppe, moyen d'apport de courant au corps du patient pour électrostimulation. Ledit dispositif d'électrostimulation 100 comporte un générateur 10 contrôlé par un calculateur 20 et des moyens logiciels 30. Ledit calculateur 20 comporte une mémoire 40 comportant des données relatives audit utilisateur prédéfinies par un examen préalable. Ledit générateur 10 comporte une pluralité de voies excitomotrices 11, chacune rattachée à un câble 12 porteur d'une pluralité de dites électrodes 1. Ledit générateur 10 est agencée pour commander l'envoi de signaux d'excitation simultanés ou séparés aux différentes voies excitomotrices 11. Chaque dit câble 12 a une longueur minimale de 150 cm. Au moins une dite électrode 1 comporte une enveloppe en élastomère thermoplastique conducteur, qui comporte au moins une puce RFID 2 agencée pour l'identification de ladite électrode 1. Au moins une dite structure porteuse 50 est souple et spongieuse, et comporte des moyens d'humidification 70. Ledit dispositif d'électrostimulation 100 comporte au moins une des structures porteuses 50 formant une ceinture ou un brassard ou un fourreau ou un manchon, ladite au moins structure porteuse 50 étant agencée pour être enfilée sur un membre ou le corps de l'utilisateur et pour porter au moins une dite électrode 1 au contact immédiat du corps de l'utilisateur. Au moins un dit câble 12 porte un nombre pair de dites électrodes 1. Au moins une dite structure porteuse 50 comporte une pluralité de logements permettant un positionnement diamétralement opposé des deux dites électrodes 1 autour d'un membre ou du corps dudit utilisateur ou/et comporte des moyens de gonflage pour son serrage autour de l'utilisateur. Au moins une dite structure porteuse 50 comporte des moyens de maintien en position autour d'un membre ou du corps dudit utilisateur, et au moins une sangle en tissu autoagrippant ; afin que les électrodes soient fixes centrées et équilibrées. Ledit générateur 10 comporte plusieurs moyens de réglage de l'intensité 60, typiquement des molettes, chacun agencé pour le contrôle de l'intensité en simultané sur au moins deux dites voies excitomotrices 11, chaque dit moyen de réglage de l'intensité 60 étant indépendant et réglable indépendamment des autres dits moyens de réglage de l'intensité 60. Dans une variante de réalisation, le boitier du dispositif 100 est prévu pour quatre boutons de réglage d'intensité, de fréquence, d'amplitude, etc. ; les huit branchements pour des câbles 12 se divisant en deux. Deux moyens sont appliqués à l'appareil, un lecteur RFID 2 et une antenne Bluetooth 3.

Le lecteur RFID 2 permet d'identifier les électrodes 1 dédiées à l'appareils et spécialement conçus pour dispenser l'amplitude et la fréquence de courant adapté

L'antenne Bluetooth 3 permets de connecter l'appareil à un logiciel

Ledit générateur 10 est agencé pour délivrer, dans chaque dite électrode 1, un courant d'intensité inférieur ou égal à 100 mA et pour gérer, pour chaque dite voie excitomotrice 11 au moins 100 trains d'impulsion de fréquence comprise entre 80 Hz et 120 Hz séparés temporellement par des phases dépourvues d'impulsion.

Chaque dite électrode 1 a une plus grande surface, agencée pour venir au contact de l'utilisateur, qui est inférieure ou égale à 100 cm².

Ledit dispositif d'électrostimulation 100 comporte un arrêt de sécurité déclenché par une horloge limitant la durée de fonctionnement à 20 minutes, ou par une action de l'utilisateur. Ledit générateur 10 comporte au moins huit dites voies excitomotrices 11 chacune reliée à au moins quatre dites électrodes 1.

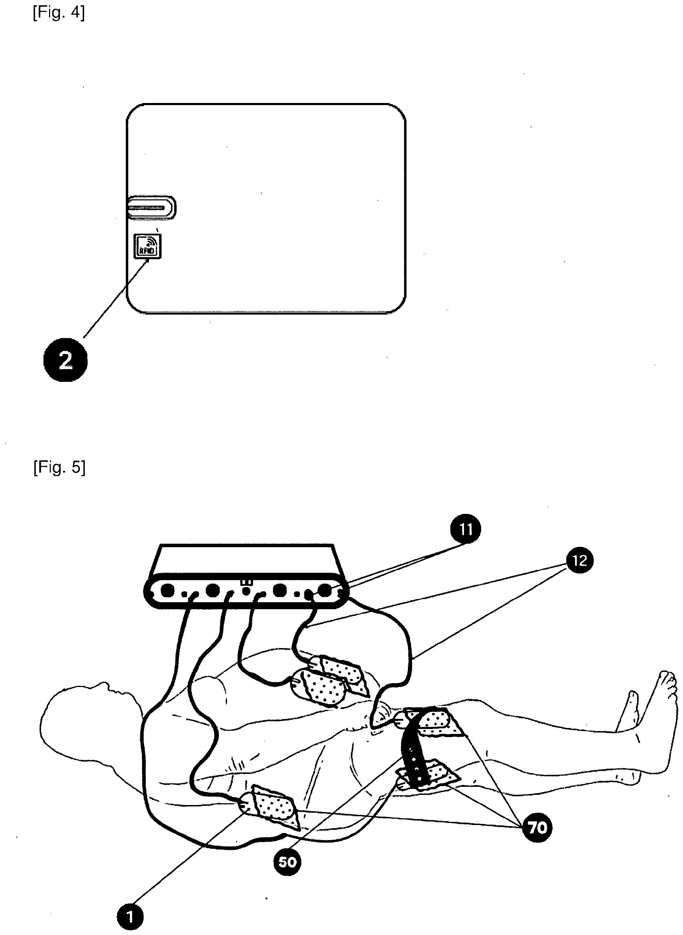

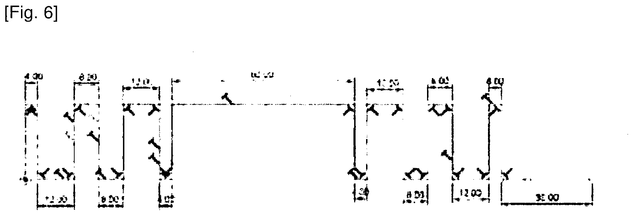

Les Figures 5 et 6 représentent en fonctionnement selon l'invention le système comprenant des électrodes d'électrostimulation 1 identiques et intervertibles branchées sur des câbles 12. Les électrodes 1 sont sur l'utilisateur pour obtenir une diminution de la masse graisseuse, produisant une électrostimulation en parallèle à la réduction calorique.

Une difficulté est que la masse musculaire protège des maladies. Elle limite l'impact de la mauvaise alimentation. Si l'amaigrissement est rapide la peau s'effondre. Ainsi, Une diminution en chute libre de la masse musculaire est pathogène.

L'objectif est d'obtenir, pendant le même temps d'électrostimulation une conversion des fibres musculaire de type 2B en type 2A, un maintien de la masse musculaire et conserver la souplesse de la peau.

Le dispositif d'électrostimulation 100 assemblé comprend le matériau en tissu des sangles permet le maintien des électrodes 1 pour assurer un placage des électrodes 1 au sens enveloppe électrodes 1 comprenant moyen conducteur noyé dans substrat de matériau plus moyen de branchement plus moyen de transmission au corps du patient. Le tout est placé dans un fourreau d'électrode en éponge mouillée optimales sur le corps du patient. Le dispositif d'électrostimulation 100 assemblé comprend encore au moins deux à quatre électrodes 1. Les électrodes 1 sont constituées en matériau TPE conducteur comprenant un élastomère thermoplastique, appelé également caoutchouc thermoplastique, au moins deux électrodes 1 étant fixées au patient outre un matériau TPE et ledit substrat corporel en outre une fibre-couche entre ledit matériau silicone conducteur et ledit matériau silicone non conducteur. Le TPE dans l'enveloppe de l'invention est conducteur, plus modelable (pour moulage), plus résistant en ohm. Il permet plusieurs plages de conductivité. Le carbone améliore la conductivité dans la fonction de polarisation.

L'inconvénient du silicone des électrodes connues est d'avoir des allergies.

Le doublement des surfaces de l'enveloppe électrode 1 répondait à une problématique (nécessité de plus d'amplitude avec la corpulence). Mais, elle se heurte à une contrainte de loi naturelle. Une surface d'électrode de 10 cm² provoque une contraction efficace et inefficace pour une surface de 100 cm². L'expérience a montré que cela n'est pas suffisant. Dans l'invention chaque câble 12 se divise pour connecter deux à quatre électrodes 1 selon la corpulence du patient. L'invention prévoie l'utilisation d'e petites moyennes électrodes 1 de taille moyenne qui sont doublées en fonction de la corpulence de l'utilisateur.

La reprise d'une activité physique tout comme la prévention des blessures repose énormément sur le bon fonctionnement du couple agoniste / antagoniste des groupes musculaires. La balance musculaire est une notion incontournable dans la prévention des blessures.

L'une des principales causes de ce déséquilibre de force musculaire est la pratique sportive elle-même. En effet, cette dernière implique la répétition de mouvements ayant pour conséquence le renforcement des muscles effecteurs (agonistes).

Les muscles antagonistes peu sollicités ne pourront donc pas évoluer sur le plan de la force au même rythme que l'agoniste sans un travail spécifique de renforcement.

La blessure antérieure toujours en ligne de mire
- L'antagoniste est un élément clé de la performance toujours très exposé dans les APS

L'antagoniste joue aussi un rôle déterminant dans tout geste sportif faisant appel à la vitesse (Jaric et coll, 1997). Si sa mise en tension n'avait pas lieu lors du geste à pleine vitesse, les dégâts sur l'articulation seraient importants et inévitables.

Par exemple au Football, les principaux muscles mis en jeu pour la jambe qui va frapper le ballon sont le psoas et les quadriceps. Les muscles antagonistes tels que les ischio-jambiers vont donner à l'articulation du genou une stabilité au moment du contact avec la balle. Cette dernière lui évitera ainsi de subir les contraintes mécaniques imposées par la réaction du ballon. Dans une autre mesure, ils permettront aussi d'éviter l'hyper extension du genou à la fin du mouvement et protégeront ainsi le ménisque et les ligaments croisés.
- Le déséquilibre musculaire au coeur de la prévention des blessures
- Le ration agoniste/antagoniste : une évidence pour le préparateur

Il semble néanmoins impossible de faire l'impasse sur le rôle du déséquilibre de force musculaire entre les muscles agonistes et antagonistes. Ce dernier peut en effet être la cause de lésions de l'appareil locomoteur, voire être à l'origine d'accidents ou de pathologies musculo-tendineuses et articulaires.

II paraît en effet difficile de penser qu'un déséquilibre de force entre le quadriceps et l'ischio- jambier soit sans conséquences sur la stabilité ou la mécanique d'une articulation telle que le genou.

Un exemple de prévention des blessures local : Le couple agoniste-antagoniste du genou

Même si la structure ligamentaire assure une grande partie de la stabilité de cette articulation, le genou subit des tensions importantes durant l'activité. Il ne peut alors la supporter qu'avec l'aide des muscles l'entourant.

Ces muscles antagonistes ont donc un rôle déterminant dans la stabilité de ce dernier. Mais leur rôle s'applique surtout dans le bon équilibre des tensions appliquées sur celui-ci. Si on prend le cas de l'épaule, cette idée est encore plus vraie. En effet, la majeure partie de sa stabilité est due à la présence de muscles forts qui entourent toute l'articulation.

De même, il existe une base théorique selon laquelle alterner les exercices musculaires antagonistes, a un effet positif sur le développement musculaire. Dans une étude menée en 2005 par le Dr Dean Baker, 24 joueurs de rugby, répartis en un groupe expérimental et un groupe témoin, ont été invités à effectuer un test de puissance sur un banc plat. Le groupe expérimental a alterné le mouvement principal avec un exercice de traction des muscles antagonistes à ceux entraînés sur un banc plat. Les résultats ont montré que la puissance musculaire avait augmenté de 4,7 % pour le groupe expérimental alors qu'aucun changement n'avait été noté pour le groupe témoin, ce qui vient renforcer l'importance d'entraîner les deux muscles du groupe antagoniste afin de développer la puissance musculaire.

Les conséquences néfastes du déconditionnement

### PHYSIOLOGIQUE

Premièrement le déconditionnement engendre une diminution de la dépense énergétique donc du tonus musculaire et de la force. Puis en parallèle une diminution des capacités aérobie (endurance fondamentale) car le coeur est aussi un muscle. Cette situation amène souvent une prise de poids (masse grasse) ayant pour effet une augmentation du risque d'hypertension.

### PSYCHOLOGIQUE

Cette sédentarité imposée peut amener à des symptômes dépressifs :
anxiété, stress, fatigue chronique, diminution de la motivation, troubles nutritionnels, dérèglement du sommeil, irritabilité... Les concentrations d'endorphine, d'adrénaline et de mélatonine sont altérées perturbant les rythmes circadiens qui nous conditionnent.

L'invention recherche l'équilibre par muscle agoniste et antagoniste.

En variante de réalisation l'appareil de l'invention présente :
a) les électrodes 1 qui sont positionnées dans des fourreaux composés de matière spongieuse (Plusieurs substrats corporels), un matériau en tissu permet de sangler les éponges 2 et les électrodes 1 sur le corps de l'utilisateur.
   Avantage de la matière spongieuse 2 : augmente la conductibilité (les éponges sont mouillées) et améliore la contraction musculaire involontaire en diminuant la contrainte de la masse graisseuse et en favorisant un meilleur contact de l'électrode 1 sur la surface du substrat corporel.
b) au moins une électrode par fourreau (substrat). Le fourreau uni électrodes 1 ou double-électrodes 1 selon la corpulence du substrat corporel

Selon une réalisation de l'invention :
a) L'invention recherche en parallèle à la restriction calorique une contraction musculaire efficace. L'EMS permet la répétition. Il est nécessaire d'avoir au moins 100 contractions. La perte de poids dépend de l'amplitude et de la fréquence. L'objectif est l'intensité la plus haute.
   L'invention a plusieurs enveloppes électrodes (électrode+ fourreau en éponge + connexion).
   Ce qui permet de s'adapter à la corpulence du patient (double les surfaces). L'éponge est souple et épouse les contours du corps.
b) Les électrodes 1 spécifiques d'une dimension de 8 cm x 12 cm qui contiennent un lecteur RFID 2 permettent de vérifier la conformité des électrodes 1. Le matériau TPE permet de contenir le lecteur RFID 2 dans l'enveloppe électrodes.
   Ce qui permet à l'appareil de s'assurer que les électrodes 1 utilisées sont bien destinées à l'usage de ce dernier. C'est une sécurité. Si non-conformité l'appareil ne fonctionne pas.
c) Le dispositif EMS 100 est en forme boîtier métallique huit voies de sortie (permettra de mettre plusieurs voies). Il est prévu de mettre un fil avec au choix deux à quatre électrodes 1.
   Ce qui permet à l'appareil de travailler sur plusieurs groupes de muscles.
   Gainer est une tétanie des muscles. Dans l'EMS stabile le patient est en position allongée.
d) Un logiciel 30 dédié qui communique avec l'appareil via la technologie Bluetooth. Soit écran affichage, antenne Bluetooth 3, quatre boutons de réglage, huit sorties un lecteur RFID 2.
f) Les électrodes 1 sont réglées identiques pour muscle agoniste et antagoniste
g) Au moins deux substrats corporels à électrodes 1 (deux fourreaux en éponges sont cousus cote à cote). Une seule électrode est placée dans un fourreau en éponge. Deux fourreaux sont cousus l'un à l'autre.

Ce qui permet d'éviter un trop grand écart ou le recouvrement entre les deux électrodes 1

L'éponge mouillée est élément conducteur qui épouse la surface corporelle. Le TPE conducteur est rigide ce qui limite les contacts avec le corps du patient.

### Plusieurs groupes de substrats

L'invention fait travailler plusieurs groupes de muscles en même temps.

L'unité électronique connectée électriquement permet la constance dans le temps, c'est-à-dire la réitération involontaire. La machine le décide. C'est la réitération de la répétition programmée d'une ou plusieurs contractions musculaires involontaires selon une intensité réglable par l'opérateur et souhaitée comme maximale grâce au courant tens combiné au courant excitomoteur dans la recherche d'un maintien ou d'une augmentation ou augmenter la masse musculaire durant une phase de recherche de perte de poids, dont une phase de restriction calorique.

Courbe EMS présente un courant excitomoteur (recherché pour la perte de masse graisseuse) et un courant Tens (réduit la douleur)

L'amplitude est variable de 0 à 120 hertz. L'efficacité commence à 85 hertz ou augmenté. Le lecteur RFID 2 permet d'identifier les électrodes 1 dédiées à l'appareils et spécialement conçues pour dispenser l'amplitude et la fréquence de courant adapté. L'antenne Bluetooth 3 permet de connecter l'appareil à un logiciel 30

L'invention recherche comme résultat de maintenir la masse musculaire. Une chute libre de cette masse musculaire est pathogène.

La masse musculaire limite l'impact de la mauvaise alimentation.

L'invention concerne un système comprenant des électrodes 1 d'électrostimulation dites EMS, leur moyen d'alimentation en particulier l'alimentation directe et la mise en oeuvre du procédé d'utilisation du système d'électrostimulation.

La base de l'invention réside dans le fait que malgré les progrès de la science, il n'existe pas à l'heure actuelle d'appareil capable de prendre en charge de façon globale, rapide et durable, la recapitalisation musculaire. Les dispositifs de l'état de la techniques (connaissance interne de la déposante) sont d'une conception ne permettant pas une prise en charge efficace notamment des personnes en surpoids ou sur certaines parties du corps. De plus, ces dispositifs restent d'une technologie complexe qui entraîne des coûts largement augmentés.

La présente invention a pour but une rupture d'ampleur dans la visée du maintien de santé et prévention de la fonte musculaire liée à l'avancée en âge. Elle rend accessible et efficace la prise en charge de personnes en surpoids ce qui concerne un nombre important de personnes en utilisant une combinaison inédite d'ultrasons et d'EMS Stabile.

L'électrostimulation est le principe d'avoir des électrodes 1 sur l'ensemble des muscles qui vont se contracter involontairement mais régulièrement et efficacement. Elle mobilise plus de 400 muscles en même temps pendant 20 minutes, avec peu ou beaucoup de mouvements selon votre niveau (pompe, gainage, squats, etc.). Pour les électrodes 1 l'invention passe en silicone et surtout "électrodes 1 type" en TPE conducteur ; modèle avec électrodes 1 à deux tailles, modèle RFID avec tailles d'électrodes 1 et logiciel intégré 30.

L'invention a pour but d'obtenir 100% du groupe musculaire contracté. Pour cela il est placé une électrode 1 par groupe musculaire/à la morphologie, obtenu un travail simultané des muscles agonistes et antagonistes, une vague d'impulsion : courant excitomoteur et courant anti-douleur (tens), utilisé les électrodes 1 connectées de puissance délivrée propre à la S² de l'électrodes 1 et utilisé des tailles d'électrodes 1 correspondant aux personnes en surpoids ou en obésité.

Le logiciel 30 dit RFID recueille les intensités et amplitudes permet une alimentation des électrodes 1 dite "type" en TPE conducteur en fonction de la corpulence du patient. Le dispositif d'électrostimulation 100 comprend des branchement (type jack) de quatre câbles de stimulation 12 identiques et intervertibles pour y brancher sur chacun quatre électrodes 1 de même taille sur fiche banane (un fil branché à deux électrodes 1). Les câbles de stimulation 12 doivent avoir la longueur suivante : longueur minimum 1m20 1m50 de façon à aller jusqu'aux membres inférieurs et supérieurs et sont compatibles avec électrodes 1.

Le procédé de mise en oeuvre du dispositif 100 entre dans objet de l'invention

Par contraste à l'invention, une activité en salle de musculation présente des inconvénients importants comme le risque de ne faire travailler qu'un seul muscle.

Or, ici, l'appareil selon l'invention permet de faire travailler le muscle agoniste et le muscle antagoniste en posant une ou plusieurs électrodes 1 sur le muscle antagoniste et le même nombre d'électrodes 1 sur le muscle antagoniste et en alimentant les électrodes 1 aux mêmes puissance et fréquence.

Le travail d'un seul muscle en salle de sport risque d'entraîner des accidents et des blessures.

De plus, la force n'est pas constante car la fatigue vient intervenir lors du travail en salle de musculation.

Or, ici, l'appareil selon l'invention permet de maintenir constante l'énergie dans les électrodes 1 sur la durée du traitement.

La nouveauté de l'invention est de s'adapter aux différents individus à des morphologies différentes.

Mais la graisse provoque des déperditions électriques, des pertes

Sachant que la masse graisseuse importante ralentit le courant, il est important de mettre plusieurs électrodes 1 plutôt que de mettre une électrode plus grande.

Plus la personne est volumineuse ; plus il est posé d'électrodes 1 :

L'appareil permet l'emploi de trente-deux électrodes 1 permet de traiter plusieurs groupes musculaires ; quatre électrodes 1 sont reliées au même branchement. L'appareil à huit sorties ou voies excitomotrices 11 de quatre branchements de quatre électrodes 1 arrive à trente-deux électrodes 1

Dans l'état de la technique actuelle, tout le circuit est au même niveau d'intensité similaire, donc il n'y a pas d'adaptabilité, de fréquence et d'amplitude homogène et pas de stabilité dans le traitement.

Si on prend un muscle agoniste comme le biceps, il n'y a pas forcément de travail sur le muscle antagoniste, le triceps en l'occurrence et nous pouvons avoir le même raisonnement avec les muscles abdominaux et dorsaux ou la cuisse avec le quadriceps et les ischio- jambier.

Comme nous l'avons vu, dans les salles de musculation on ne travaille parfois qu'un seul de ces muscles, nous avons un risque de blessure. L'avantage de l'électrostimulation selon l'invention est d'arriver à être homogène, ce qui dans l'état de la technique peut au mieux être fait partiellement.

Il n'est pas possible de dépasser 100 milliampères pour une personne.

L'appareil, est visuellement un boitier. Ce boîtier est muni d'un lecteur RFID 2 et un connecteur Bluetooth, le connecteur Bluetooth présente une antenne extérieure 3.

Ce boîtier a une fonction écologique ; sa forme est importante, elle évite d'avoir un moule en plastique et elle permet l'évolution de l'appareil sans modifier sa structure. Dans ce boîtier métallique, il reste de la place, ce qui a permis de mettre au minimum huit voies de sortie et il permettra de mettre plusieurs voies. Il peut recevoir des fils avec respectivement quatre électrodes 1. Donc, la fréquence et l'amplitude sont identiques, ce qui permet d'obtenir l'homogénéité de l'intensité et un travail sur les muscles agonistes et antagonistes. Le travail doit se faire avec au moins deux électrodes 1, c'est-à-dire au minimum en mettre une au-dessus et une en dessous.

L'appareil selon l'invention permet de faire travailler le muscle agoniste et le muscle antagoniste en posant une ou plusieurs électrodes 1 sur le muscle antagoniste et le même nombre d'électrodes 1 sur le muscle antagoniste et en alimentant les électrodes 1 aux mêmes puissance et fréquence.

Le travail d'un seul muscle en salle de sport risque d'entraîner des accidents et des blessures. De plus, la force n'est pas constante car la fatigue vient intervenir lors du travail en salle de musculation.

Par contraste, l'appareil selon l'invention permet de maintenir constante l'énergie dans les électrodes 1 sur la durée du traitement.

L'appareil permettant l'emploi de trente-deux électrodes 1 permet de traiter plusieurs groupes musculaires, quatre électrodes 1 sont reliées au même branchement

Un avantage de l'invention est de s'adapter aux différents individus à des morphologies différentes. La graisse provoque des déperditions électriques, des pertes, une masse graisseuse importante ralentit le courant, il est important de mettre plusieurs électrodes 1 plutôt que de mettre une électrode plus grande.

L'appareil à huit sorties de quatre branchements de quatre électrodes 1 ; soit trente-deux électrodes 1 permet de poser plus d'électrodes 1, plus la personne est volumineuse,

Dans l'état de la technique actuelle, tout le circuit est au même niveau d'intensité similaire, donc il n'y a pas d'adaptabilité, de fréquence et d'amplitude homogène et pas de stabilité dans le traitement. Si on prend un muscle agoniste comme le biceps, il n'y a pas forcément de travail sur le muscle antagoniste, le triceps en l'occurrence et nous pouvons avoir le même raisonnement avec les muscles abdominaux et dorsaux. Dans les salles de musculation on ne travaille parfois qu'un seul de ces muscles, nous avons un risque de blessure. L'avantage de l'électrostimulation selon l'invention est d'arriver à être homogène.

Il n'est pas possible de dépasser 100 milliampères pour une personne.

L'invention a pour objet d'arriver à dispenser une remise en forme globale combinant nutrition physique et physiologique. Les problématiques abordées sont basées sur les thématiques de la gestion du poids, la prévention santé, et le « bien vieillir ». Le système selon l'invention s'insère dans l'accompagnement de nombreuses pathologies : pathologies chroniques, diabète de type 2, surpoids / obésité, stéatose hépatique (NASH) circulation sanguine, dyslipidémie, cardiovasculaires, apnée du sommeil, hormonaux, graisses localisées, morphologies, prévention du vieillissement (sarcopénie), poste grossesse, post chirurgies bariatriques, addictions. Renforcement musculaire.

La structure du système de l'invention est illustrée dans ce qui suit. Développement de l'appareil d'électrostimulation. Adaptation à la surface corporelle de : l'intensité, la fréquence et le montage des électrodes d'électrostimulation (qui elle-même est adaptée au public spécifique visé). Les extensions des dispositifs du système selon l'invention peuvent se connecter entre eux pour s'adapter à la morphologie de la personne visée. Le système selon l'invention obtient cette adaptation avec différentes électrodes 1. D'où le système comprend différentes versions de câbles 12 pour s'adapter aux différentes électrodes 1.

Les électrodes 1 sont en silicone et sont surtout des électrodes 1 "type" en TPE conducteur. Même s'il est utilisé différentes versions de boîtiers d'alimentation en dehors de la marque qui sera imprimé sur l'appareil, le boîtier sera le même.

Le système de stimulation selon l'invention comprend différentes versions de dispositifs100, soit modèle avec électrodes 1 à deux tailles ou modèle RFID avec trois tailles d'électrodes 1 qui sont équipées d'un logiciel 30 intégré.

Avec un logiciel 30 propre aux électrodes 1. L'évaluation des tests existants :
- Prise de données toutes les secondes (en temps réel) pour adapter les courbes aux besoins du patient. Développement d'un appareil à ultrason. Deux caractéristiques : Intensité toujours homogène.

Un dispositif 100 exigences fonctionnelles Unité huit canaux : Se compose d'une unité de stimulation disposant de quatre canaux alimentant chacun quatre électrodes 1. Spécifications électriques : Être alimenté via une prise conforme CE vers le secteur 100-240V 50/60Hz. Connectiques câbles 12 : Recevoir les branchement (type jack) de quatre câbles de stimulation 12 identiques et intervertibles pour y brancher sur chacun quatre électrodes 1 de même taille sur fiche banane (1 fil = 2 électrodes 1). Les câbles de stimulation 12 doivent avoir la longueur suivante : longueur minimum 1m 50 de façon à aller jusqu'aux membres inférieurs et supérieurs. Les électrodes 1 standards sont compatibles avec des électrodes 1 conformes CE dédiées à l'EMS à coller sur la peau de l'utilisateur zones de positionnements selon les points indiqués : - Classique femme - Classique homme - gynoïde - androïde - triceps

Dans le système selon l'invention, l'unité de stimulation est compatible avec les électrodes 1 silicone Graphite dit GymnaUniphy, 80 x 120 mm avec fiche banane femelle 2mm + adaptateur 4mm pour pallier à la fragilité.

Le procédé selon l'invention de la mise en oeuvre du mode opératoire ci-dessous comprend les étapes suivantes : - connecter adaptateur secteur, - choisir électrodes 1 ;- connecter cordons électrodes 1, - connecter électrodes 1, - positionner électrodes 1 sur zone peau utilisateur, - mettre unité sous tension (ON/OFF), - sélectionner durée traitement, - augmenter progressivement l'énergie de sortie (intensité) pour atteindre la valeur adaptée, - attendre fin du temps programme (arrêt), - éteindre appareil, - retirer électrodes 1, - débrancher et ranger les éléments.

Utilisation principale de la phase d'exploitation du procédé : - connecter adaptateur secteur - connecter cordons électrodes 1 - connecter électrodes 1 - positionner électrodes 1 sur zones peau utilisateur - mettre unité sous tension (ON/OFF) - sélectionner durée traitement - augmenter progressivement l'énergie de sortie (intensité) pour atteindre la valeur adaptée - attendre fin du temps programme (arrêt) - éteindre appareil - retirer électrodes 1 - débrancher et ranger les éléments,

En vue d'électrostimulation des muscles, le système de stimulation électrique musculaire intègre un logiciel EMS 30. Dans l'invention des appareils d'EMS stabile / involontaire sont utilisés dans le cadre des cures de remise en forme et de perte de poids de ses clients.

Selon l'invention le dispositif d'EMS stabile / involontaire 100 est utilisé dans le cadre de soins à visée esthétique afin de maintenir ou développer la masse musculaire sur les zones sous développées ou en décroissance du corps du client adulte, tout en diminuant la masse graisseuse et en améliorant la texture de la peau (cellulite et raffermissement) durant la perte de poids.

Un but de l'invention est d'envoyer des impulsions électriques aux muscles de zones ciblées pour les stimuler durant des séances de durée prédéfinie.

Les Objectifs : (ce que le système doit faire et ses performance, conditions et contraintes éventuelles) - appareils d'électrostimulations huit voies pour seize électrodes 1, pour un total de seize appareils ; - fonctionnant avec des consommables avec, par la suite en option, électrodes 1 à taille spécifiques - Touches manuelles - un fil branche deux électrodes 1 (2 mm banane) - les paires (canaux) sont gérées quatre par quatre donc quatre boutons de gestion (augmenter de façon homogène, plutôt bouton +/-) + un bouton principal marche/arrêt + affichage intensité par chiffre ou graphique (pas d'écran tactile) écran TFT couleur gen4-ulcd-5DD1 ou barre graph ; - boitier plutôt large - long pour positionnement ; - intensité mesurée : prévoir plus haut (nouvelle génération) pour certains clients.

Définition du contexte du dispositif de stimulation 100 selon l'invention :
Le schéma des phases de vie du dispositif de stimulation 100, c'est à dire le processus couvrant les phases de sa création jusqu'à sa disparition (ex : besoins, exigences, conception, développement, vérification, validation, installation, exploitation, maintenance/mises à jour, fin de vie ...). Conception - développement - Industrialisation - Essais de sécurité - Marquage CE - Production (- Contrôle - Etiquetage - Emballage - Libération) - Stockage - Transport - Formation - Utilisation Maintenance - Mise au rebut

Le dispositif de stimulation 100 est adapté aux éléments du milieu extérieur (autres que les personnes) qui peuvent influer sur le système (équipements : technologies, énergies, sol, implantation ..., matières : entrées et sorties de matières, fluides ..., environnement : poussière, chaleur, retraitement des déchets ..., estime : coûts, esthétique, ...) : - réseau électrique 100-240VA 50/60Hz - électrodes 1 (x4 packs de 4) - peau de l'utilisateur - environnement centre de remise en forme - esthétique - possibilité d'usage (interface boutons) - réglementations et normes CE.

Pour chaque phase de vie où des services du dispositif 100 sont attendus (ex : phase d'exploitation / utilisation et phase de maintenance), au regard des cas d'utilisation (séquences d'utilisation) et les parties prenantes concernées. La mission principale du système se trouve dans le cas d'utilisation principal de la phase d'exploitation. A savoir, l'utilisation (séance d'électrostimulation des muscles) : - connecter adaptateur secteur - connecter cordons électrodes 1 - connecter électrodes 1 - positionner électrodes 1 sur zones peau utilisateur - mettre unité sous tension (ON/OFF) - sélectionner durée traitement - augmenter progressivement l'énergie de sortie (intensité) pour atteindre la valeur adaptée - attendre fin du temps programmé (arrêt) - éteindre appareil - retirer électrodes 1 - débrancher et ranger les éléments.

Pour chaque cas d'utilisation identifié, le scénario principal d'utilisation du dispositif 100 (et alternatif le cas échéant), c'est à dire les séquences d'actions de l'utilisateur, d'une autre partie prenante ou du système (entrées et sorties du système) permettant de réaliser le cas d'utilisation, d'est-à-dire le « où » et le « quand », ou faire référence au document concerné.

Le scénario varie en fonction de : - sexe de l'utilisateur (influant sur les zones à stimuler) ; - corpulence (influant sur la taille des électrodes 1) ; - zones stimulées (les points sont connus par l'inventeur) ; - durées de séance ; - intensité de sortie.

Chaque cas d'utilisation du dispositif 100 entraine des besoins (les objectifs associés au cas d'utilisation / exigence fonctionnelle, en termes de performances, opérationnel, d'interfaces cf. : typologies d'exigences non fonctionnelles ci-dessous) :

Dans les essais, les besoins de contraintes (pour chaque élément extérieur, le contexte a entrainé les contraintes de cet élément) sont présentés à partir des diagrammes d'environnement / de contexte de type pieuvre.

Le dispositif de stimulation 100 répond aux exigences de se composer d'une unité de stimulation disposant de quatre canaux alimentant chacun quatre électrodes 1 ; est alimenté via une prise conforme CE vers le secteur 100-240V 50/60Hz ; reçoit les branchement (type jack) de quatre câbles de stimulation 12 identiques et intervertibles pour y brancher sur chacun quatre électrodes 1 de même taille sur fiche banane (un fil = deux électrodes 1). Les câbles de stimulation 12 doivent avoir la longueur suivante : longueur minimum 1m20 de façon à aller jusqu'aux membres inférieurs et supérieurs. Le dispositif 100 est compatible avec des électrodes 1 conformes CE dédiées à l'EMS à coller sur la peau de l'utilisateur zones de positionnements (points fournis par client) : - Classique femme - Classique homme - Gynoïde - Androïde - Triceps.

Le dispositif de stimulation 100 est compatible avec des électrodes 1 silicone graphité, 80 x 120 mm avec fiche banane femelle 2mm + adaptateur 4mm pour pallier à la fragilité. Elle dispose d'un système de commande manuel comprenant :
- quatre molettes « + / - » de gestion d'intensité des impulsions électriques 60
- un bouton principal marche/arrêt (alimentation ON/OFF)
- un bouton de sélection de durée du traitement.

Le dispositif de stimulation 100 dispose d'un système d'affichage couleur gen4-ulcd-5DD1 comprenant un barre-graphe LED et afficheur sept segments. Elle est protégée contre l'ouverture par des tiers non autorisés (ne doit pas pouvoir être ouverte sans utiliser des outils spécifiques). Le dispositif 100 dispose d'un système de protection contre les surintensités.

Les spécifications EMS suivantes sont réalisées (mesures effectuées sur appareil avec électrode silicone Graphité GymnaUniphy, 80 x 120 mm avec fiche banane femelle 2mm) :
- puissance sortie des impulsions / canal = 90 V x 80mA = 7,2 VA (ou W)
- fréquence de sortie unique = 2,7kHz
- durées d'impulsions
- forme d'impulsions = carrée
- type d'impulsion = compensées

Le dispositif de stimulation 100 permet de régler la durée de sortie (séance d'EMS) en appuyant sur le bouton + / -, par pas de 1 minute, de 0 à 99 minutes - d'empêcher la modification du timer après le lancement de la séance d'EMS. Dans ce cas, il est possible de terminer la séance en réduisant l'énergie de sortie jusque 0 puis en éteignant le bouton d'alimentation (OFF). Sinon, la stimulation s'arrête automatiquement à la fin du minuteur. L'intensité de sortie doit être réglée, pour chaque chacun des quatre canaux, à l' aide d'une molette - / + par pas de 1 % de la valeur maximum (90 V x 80mA = 7,2 VA (ou W)) de 0 à 100 % (conforme à la norme EN 60601-2-10 → 201.12.1.101 Le pas de réglage ne doit pas excéder 1mA ou 1V et au réglage minimal, ne pas excéder 2 % de la valeur disponible au réglage max). La sortie d'EMS peut être stoppée par un maintien de l'appuie sur la molette de réglage d'intensité du canal 60 (appui en continue pendant une seconde au minimum). L'intensité de sortie doit être limitée conformément au § 201.12.4.104 de la norme EN IEC 60601-2-10. Le dispositif 100 affiche la durée de séance restante en minutes. Il afficher le niveau de sortie EMS au 100%age du niveau de sortie maximum dans un graphique à barres. Le dispositif 100 avertit lorsque le courant de sortie dépasse une valeur de sécurité spécifiée. Exemple : « OVERCURRENT » s'affiche et clignote. Il disparaît quand l'intensité est diminuée mais le système s'éteint dans les trois secondes si aucune action n'est réalisée. Pour poursuivre la séance, il faut éteindre le système puis le rallumer. Le dispositif 100 avertit lorsque l'électrode ou le cordon d'électrode n'est pas correctement connecté pendant la sortie. Le dispositif 100 dispose d'indicateurs sonores pour : - signaler la fin du timer. Il dispose d'indicateurs de codes erreurs :
- manque de tension sur l'alimentation ;
- excès de tension sur l'alimentation ;
- problème de circuit.

Le dispositif de stimulation 100 est utilisé selon le procédé qui comprend les étapes suivantes : - connecter adaptateur secteur - connecter cordons électrodes 1 - connecter électrodes 1 - positionner électrodes 1 sur zone peau utilisateur - mettre unité sous tension (ON/OFF) - sélectionner durée traitement - augmenter progressivement l'énergie de sortie (intensité) pour atteindre la valeur adaptée - attendre fin du temps programmé (arrêt) - éteindre appareil - retirer électrodes 1 - débrancher et ranger les éléments.

Le dispositif de stimulation 100 est conforme aux normes de sécurité applicables zone UE (cf. cadrage réglementaire « Cadrage réglementaire _HTC_EMS_V1 2021-12-22 ») et disposer d'un marquage CE selon les réglementations applicables zone UE (cf. cadrage réglementaire).

Suivant d'autres caractéristiques du dispositif de stimulation 100 de l'invention le logiciel 30 dit RFID permet une alimentation des électrodes 1 dite "type" en TPE conducteur en fonction de la corpulence du patient. Le dispositif 100 comprend des branchement (type jack) de quatre câbles de stimulation 12 identiques et intervertibles pour y brancher sur chacun quatre électrodes 1 de même taille sur fiche banane (1 fil = 2 électrodes 1). Les câbles de stimulation 12 ont la longueur suivante : longueur minimum 1m20 1m50 de façon à aller jusqu'aux membres inférieurs et supérieurs et sont compatibles avec électrodes 1. Les électrodes 1 sont équipées d'un lecteur RFID 2, à savoir avec un scanner par appareil. Le tout connecté au boîtier. Ces électrodes 1 sont de tailles à s'adapter à la corpulence du patient. Le courant est uniforme mais s'adapte à la morphologie, à savoir que suivant la corpulence du patient, avec des électrodes 1 identiques qui ont une fonction de lipolyse, les résultats peuvent être différents.

Pour remédier à ces variations de résultats, des électrodes 1 (connectées au boîtier) sont de tailles différentes et la fréquence est adaptée par rapport aux électrodes 1 en question pour réussir à obtenir un effet standard quel que soit le patient.

Le procédé de mise en oeuvre du mode opératoire ci-dessous avec un système typiquement conforme à celui décrit précédemment comprend les étapes suivantes : - connecter adaptateur secteur ; - choisir électrodes 1 ; - connecter cordons électrodes 1 ; - connecter électrodes 1 ; - positionner électrodes 1 sur zone peau utilisateur ; - mettre unité sous tension (ON/OFF) ; - sélectionner durée traitement ; - augmenter progressivement l'énergie de sortie (intensité) pour atteindre la valeur adaptée ; - attendre fin du temps programme (arrêt) ; - éteindre appareil - retirer électrodes 1 ; - débrancher et ranger les éléments.

Conforme aux normes de sécurité applicables zone UE (cf. cadrage réglementaire « Cadrage réglementaire _HTC_EMS_V1_2021 -12-22 »)

Disposer d'un marquage CE selon les réglementations applicables zone UE (cf. cadrage réglementaire).

Position du problème de la phase d'amaigrissement sans contrôle et maintien de la masse musculaire : La prise en charge du surpoids et de l'obésité vise à plusieurs objectifs : la perte de poids, puis la stabilisation pondérale. En effet, l'intérêt de l'électrostimulation peut être considéré comme une nouvelle voie de prise en charge pour le maintien de cette masse musculaire dans la phase de restriction calorique. Le souci étant qu'actuellement il n'existe pas d'appareil en dispositif médical en électrostimulation stabile (sans mouvement) adapté aux personnes en surpoids ou en obésité. Pas perte de muscle activité physique problème : ne perdre que la masse grasse.

L'invention surmonte les contre-indications sportives pour un utilisateur obèse et les phase de restriction calorique.

### EMS

Le plus efficace est une combinaison. La musculation demande de transporter un poids supplémentaire au poids naturel du corps et une activité intense.

L'avantage de l'EMS est d'éviter les contraintes de l'activité musculaire classique.

Un muscle gras n'a pas la même puissance qu'un muscle de qualité avec fibres rapides. D'où nécessité d'entretenir l'ensemble des groupes musculaires.

L'invention a pour domaine le renforcement musculaire.

Ce n'est pas uniquement le volume, mais la qualité du muscle, par un renforcement musculaire intense homogène qui est efficace. L'EMS est mesurable. Marcher n'a pas d'effet bénéfique car le corps est destiné à marcher. Le bénéfice commence selon le niveau d'intensité. Pour obtenir ce bénéfice il faut aller très loin, le patient ne peut plus suive.

Maintien de la peau. C'est une question de tissu épitémiade. La contraction oxygène les tissus. C'est un drainage.

L'Installation de culture physique selon l'invention comporte au moins un agrès ou une machine d'exercice musculaire ou un tapis d'exercice, et comportant au moins un dispositif d'électrostimulation 100 selon la description ci-dessus.

L'Installation de détente selon l'invention comporte un siège ou une couche pour supporter l'utilisateur, et comportant au moins un dispositif d'électrostimulation 100 selon la description ci-dessus. Bien que l'invention ait été décrite en liaison avec des structures particulières, elle n'y est nullement limitée et l'on pourra y apporter de nombreuses variantes de réalisation il sera évident qu'elle peut être modifiée de nombreuses manières. De telles variantes ne doivent pas être considérées comme s'écartant de l'esprit de l'invention. Toutes les modifications qui seraient évidentes pour un homme du métier sont destinées à être incluses dans la portée des revendications qui suivent.

Les combinaisons des différentes réalisations représentées sur les figures ou décrites ci-dessus ne sortent pas du cadre de l'invention.

### Signes de référence

- 1: Electrodes
- 2.: Puce RFID
- 3.: Antenne
- 10: Générateur
- 11: Voies excitomotrices
- 12: Câble
- 20: Calculateur
- 30: Moyens logiciels
- 40: Mémoire
- 50: Structure porteuse
- 60: Moyens de réglage de l'intensité
- 70: Moyens d'humidification
- 100: Dispositif d'électrostimulation

Les signes de référence insérés après les caractéristiques techniques mentionnées dans les revendications ont pour seul but de faciliter la compréhension de ces dernières et n'en limitent aucunement la portée.

## Revendications

1. Dispositif d'électrostimulation (100) agencé pour l'électrostimulation sur un utilisateur de forte corpulence, ayant un indice de masse corporelle supérieur à 26 à 32 kg/m², un tour de taille supérieur à 108 cm, une masse supérieure à 100 kg, ledit dispositif d'électrostimulation (100) comportant une pluralité d'électrodes (1), **caractérisé en ce que** ledit dispositif d'électrostimulation (100) comporte un générateur (10) contrôlé par un calculateur (20) et des moyens logiciels (30), ledit calculateur (20) comportant au moins une mémoire (40) comportant des données relatives audit utilisateur prédéfinies par un examen préalable, ledit générateur (10) comportant une pluralité de voies excitomotrices (11), chacune rattachée à un câble (12) porteur d'une pluralité de dites électrodes (1), et étant agencée pour commander l'envoi de signaux d'excitation simultanés ou séparés aux différentes voies excitomotrices (11).

2. Dispositif d'électrostimulation (100) selon la revendication (1), **caractérisé en ce que** chaque dit câble (12) a une longueur minimale de 120 cm.

3. Dispositif d'électrostimulation (100) selon la revendication 1 ou 2, **caractérisé en ce qu'**au moins une dite électrode (1) comporte une enveloppe en élastomère thermoplastique conducteur, qui comporte au moins une puce RFID (2) agencée pour l'identification de ladite électrode (1).

4. Dispositif d'électrostimulation (100) selon l'une des revendications précédentes, **caractérisé en ce qu'**au moins une dite structure porteuse (50) est souple et spongieuse, et comporte des moyens d'humidification (70).

5. Dispositif d'électrostimulation (100) selon la revendication 4, **caractérisé en ce que** ledit dispositif d'électrostimulation (100) comporte au moins une des structures porteuses (50) formant une ceinture ou un brassard ou un fourreau ou un manchon, ladite au moins structure porteuse (50) étant agencée pour être enfilée sur un membre ou le corps de l'utilisateur et pour porter au moins une dite électrode (1) au contact immédiat du corps de l'utilisateur.

6. Dispositif d'électrostimulation (100) selon l'une des revendications 1 à 5, **caractérisé en ce qu'**au moins un dit câble (12) porte un nombre pair de dites électrodes (1).

7. Dispositif d'électrostimulation (100) selon les revendications 5 et 6, **caractérisé en ce qu'**au moins une dite structure porteuse (50) comporte une pluralité de logements permettant un positionnement diamétralement opposé des deux dites électrodes (1) autour d'un membre ou du corps dudit utilisateur ou/et comporte des moyens de gonflage pour son serrage autour de l'utilisateur.

8. Dispositif d'électrostimulation (100) selon la revendication 5 et l'une des revendications 1 à 7, **caractérisé en ce qu'**au moins une dite structure porteuse (50) comporte des moyens de maintien en position autour d'un membre ou du corps dudit utilisateur, et au moins une sangle en tissu autoagrippant.

9. Dispositif d'électrostimulation (100) selon l'une des revendications 1 à 8, **caractérisé en ce que** ledit générateur (10) comporte plusieurs moyens de réglage de l'intensité (60), typiquement des molettes, chacun agencé pour le contrôle de l'intensité en simultané sur au moins deux dites voies excitomotrices (11), chaque dit moyen de réglage de l'intensité (60) étant indépendant et réglable indépendamment des autres dits moyens de réglage de l'intensité (60).

10. Dispositif d'électrostimulation (100) selon l'une des revendications 1 à 9, **caractérisé en ce que** ledit générateur (10) est agencé pour délivrer, dans chaque dite électrode (1), un courant d'intensité inférieur ou égal à 100 mA et pour gérer, pour chaque dite voie excitomotrice (11) au moins 100 trains d'impulsion de fréquence comprise entre 80 Hz et 120 Hz séparés temporellement par des phases dépourvues d'impulsion.

11. Dispositif d'électrostimulation (100) selon une des revendications 1 à 10, **caractérisé en ce que** chaque dite électrode (1) a une plus grande surface, agencée pour venir au contact de l'utilisateur, qui est inférieure ou égale à 100 cm².

12. Dispositif d'électrostimulation (100) selon l'une des revendications 1 à 11, **caractérisé en ce que** ledit dispositif d'électrostimulation (100) comporte un arrêt de sécurité déclenché par une horloge limitant la durée de fonctionnement à 20 minutes, ou par une action de l'utilisateur.

13. Dispositif d'électrostimulation (100) selon l'une des revendications 1 à 12, **caractérisé en ce que** ledit générateur (10) comporte au moins huit dites voies excitomotrices (11) chacune reliée à au moins quatre dites électrodes (1).

14. Installation de culture physique, comportant au moins un agrès ou une machine d'exercice musculaire ou un tapis d'exercice, et comportant au moins un dispositif d'électrostimulation (100) selon une des revendications 1 à 13

15. Installations de détente, comportant un siège ou une couche pour supporter l'utilisateur, et comportant au moins un dispositif d'électrostimulation (100) selon une des revendications 1 à 13. 1
